# FASCICULE DE BREVET EUROPEEN

(11) **EP 4 507 771 B1**
(45) Date de publication et mention de la délivrance du brevet: **17.12.2025**
(21) Numéro de dépôt: 23714771.5
(22) Date de dépôt: 07.04.2023
(51) Int. Cl.: A61M 39/22, F16K 5/04, F16K 11/085

(54) **ROBINET MÉDICAL**
MEDIZINISCHES VENTIL
MEDICAL VALVE

(30) Priorité: 11.04.2022 FR 2203287
(43) Date de publication de la demande: 19.02.2025
(73) Titulaire: Guerbet, 93420 Villepinte (FR)
(72) Inventeur: ALLARD, Ludovic, 69390 Millery (FR)
(74) Mandataire: Lavoix
(86) Numéro de dépôt international: PCT/EP2023/059314
(87) Numéro de publication internationale: WO 2023/198644

(56) Documents cités:
- WO-A1-2016/166346
- FR-A1- 3 034 998
- US-A- 4 807 666

## Description

La présente invention concerne un robinet médical.

L'invention concerne notamment, mais non exclusivement, les robinets médicaux utilisés en radiologie interventionnelle.

L'invention s'intéresse aux robinets médicaux comprenant un corps creux, qui est muni d'au moins trois ports et à l'intérieur duquel est monté rotatif un boisseau qui commande la mise en communication à travers lui entre au moins deux des ports du corps du robinet. La grande majorité des robinets de ce type, présents sur le marché, sont faits pour des usages mettant en œuvre des pressions inférieures à 3 bars, par exemple pour les besoins d'une perfusion. Ceci étant, pour certains usages, les robinets médicaux utilisés sont soumis à des pressions bien plus fortes, pouvant dépasser les 50 bars et atteindre voire dépasser les 100 bars. C'est notamment le cas en radiologie interventionnelle, par exemple pour réaliser une chimioembolisation transartérielle ultra-sélective, couramment appelée cTACE, où des micro cathéters sont utilisés pour injecter au plus près de tumeurs, en passant par des vaisseaux de petites tailles, des mélanges visqueux comprenant des solutions aqueuses et des huiles, ce qui nécessite de pousser ces mélanges avec des pressions pouvant monter aux hautes valeurs précitées. WO 2016/166346 divulgue un exemple d'un robinet médical adapté à ce contexte d'utilisation. US-4 807 666-A1 décrit un autre exemple de robinet médical de l'art antérieur.

Plus les pressions d'utilisation sont élevées, plus les robinets médicaux présentent un risque de fuite, en particulier au niveau de l'interface, normalement étanche, entre le corps et le boisseau du robinet. Pour renforcer cette étanchéité, une opinion communément répandue consiste à augmenter l'interférence de contact, notamment l'emmanchement, entre le corps et le boisseau. Cette approche conduit toutefois à augmenter considérablement le couple nécessaire au déplacement rotatif du boisseau, ce qui rend le robinet peu pratique à utiliser.

Le but de la présente invention est de proposer un robinet médical amélioré dont la résistance aux fuites est renforcée, sans augmenter significativement les contraintes d'utilisation du robinet, notamment du couple nécessaire au déplacement rotatif du boisseau.

À cet effet, l'invention a pour objet un robinet médical, tel que défini à la revendication 1.

En opposition au préjugé technique selon lequel on ne peut agir efficacement sur la résistance aux fuites qu'en augmentant le diamètre extérieur du boisseau pour accroître l'interférence de contact entre le corps de robinet et le fût du boisseau du robinet, les inventeurs ont mis en évidence qu'il était plus intéressant, non pas d'augmenter l'interférence de contact, mais de maintenir l'interférence tout en augmentant l'épaisseur du fût moyennant une réduction du diamètre intérieur du fût. De plus, plutôt que d'augmenter ainsi l'épaisseur du fût sur toute la périphérie du fût, les inventeurs ont également mis en évidence qu'il était bien plus intéressant de surépaissir intérieurement le fût uniquement dans la portion de la paroi tubulaire de ce fût, où sont situées les ouvertures par lesquelles le canal intérieur au boisseau débouche sur la surface latérale extérieure de cette paroi tubulaire. Cela induit de décentrer le diamètre intérieur du fût par rapport à son diamètre extérieur. L'invention prévoit ainsi de surépaissir intérieurement le fût, et donc d'augmenter la raideur de ce dernier, exclusivement dans sa portion précitée, où s'exerce le plus de pression lors de l'utilisation du robinet médical, tandis que la portion diamétralement opposée du fût n'est pas surépaissie, ce qui permet de répartir le champ de pression de manière plus homogène et minimiser le risque de fuite à l'extérieur de l'interface entre le boisseau et le corps du robinet. Grâce à l'invention, le robinet médical résiste à des pressions élevées, notamment supérieures à 65 bars et pouvant aller jusqu'à 80 bars, voire 100 bars, sans apparition de fuite, tout en restant facile à manipuler. En pratique, comme détaillé par la suite, la surépaisseur intérieure localisée du fût peut présenter divers aménagements avantageux et/ou être associée à un renforcement de l'épaisseur du canal et à un renforcement d'une cloison de maintien de ce canal, notamment en vue d'améliorer encore les performances d'étanchéité du robinet médical conforme à l'invention.

Des caractéristiques additionnelles avantageuses du robinet médical conforme à l'invention sont spécifiées aux autres revendications.

L'invention sera mieux comprise à la lecture de la description qui va suivre, donnée uniquement à titre d'exemple et faite en se référant aux dessins sur lesquels :
- la figure 1 est une vue en perspective d'un robinet médical conforme à l'invention ;
- la figure 2 est une coupe selon le plan II de la figure 1 ;
- la figure 3 est une vue en perspective d'un premier mode de réalisation d'un boisseau du robinet médical des figures 1 et 2 ;
- la figure 4 est une coupe dans le plan IV de la figure 3 ;
- la figure 5 regroupe des inserts A), B) et C) correspondant respectivement à des sections selon les lignes A, B et C de la figure 4 ; et
- la figure 6 est une vue similaire à la figure 5, illustrant un second mode de réalisation du boisseau.

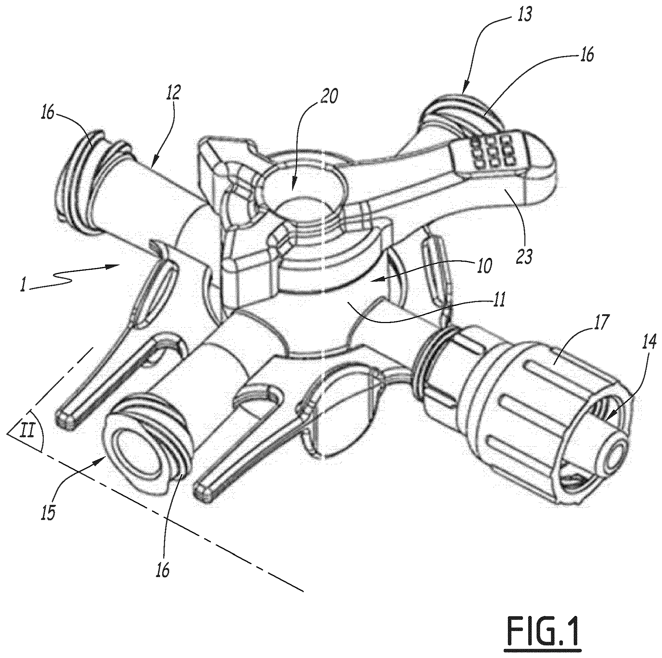

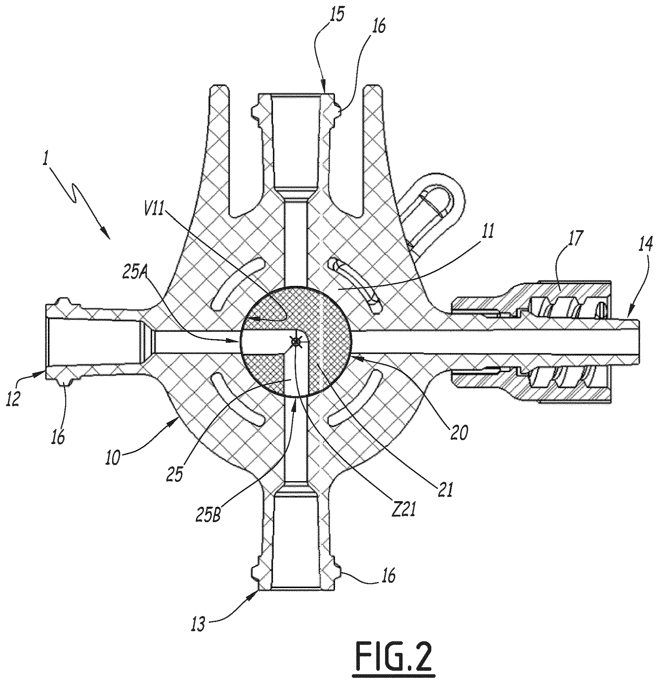

Sur les figures 1 et 2 est représenté un robinet médical 1. Ce robinet médical 1 est notamment, mais non exclusivement, utilisable en radiologie interventionnelle, notamment pour réaliser une chimioembolisation transartérielle, communément appelée cTACE, le cas échéant ultra-sélective, c'est-à-dire réalisée au niveau de la partie la plus distale de la vascularisation d'une tumeur. Le robinet médical 1 est notamment utilisable dans le contexte et selon la méthode, qui sont détaillés dans WO2016/166346 auquel le lecteur peut se reporter pour connaître les spécificités correspondantes du robinet médical 1.

Dans tous les cas, le robinet médical 1 comprend un corps 10 et un boisseau 20.

Le corps 10 comporte un fût 11 tubulaire, qui, comme montré sur la figure 2, définit un volume interne V11 à l'intérieur duquel est reçu le boisseau 20, comme détaillé par la suite.

Le corps 10 comporte également quatre ports 12, 13, 14 et 15, qui s'étendent chacun depuis le fût 11 à l'opposé du volume interne V11 et qui sont ici régulièrement répartis autour de la périphérie extérieure du fût 11. Chaque port 12, 13, 14, 15 relie le volume interne V11 à l'extérieur du corps 10, via un conduit qui traverse le fût 11 transversalement de part en part et qui s'étend à l'intérieur du port correspondant suivant une direction longitudinale de ce dernier. Chaque port 12, 13, 14, 15 forme un connecteur permettant de raccorder le corps 10 à un matériel médical adapté. Dans l'exemple envisagé sur les figures, les ports 12, 13 et 14 forment respectivement des connecteurs femelles qui sont ici pourvus chacun d'un filetage externe 16 à même de recevoir par vissage, par exemple, un luer ; le port 15 forme, quant à lui, un connecteur mâle, préférentiellement un connecteur luer, qui est ici équipé d'une bague de verrouillage 17. Ceci étant, les spécificités des ports 12 à 15, relatives à leur capacité de raccordement à des matériels médicaux utilisables avec le robinet médical 1, ne sont pas limitatives. De même, chacun des ports 12 à 15 peut être utilisé indifféremment en port d'entrée ou en port de sortie, selon le contexte d'utilisation du robinet médical 1.

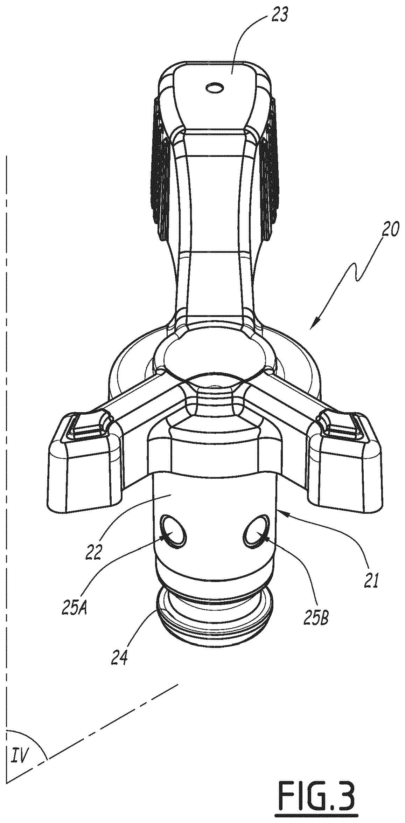

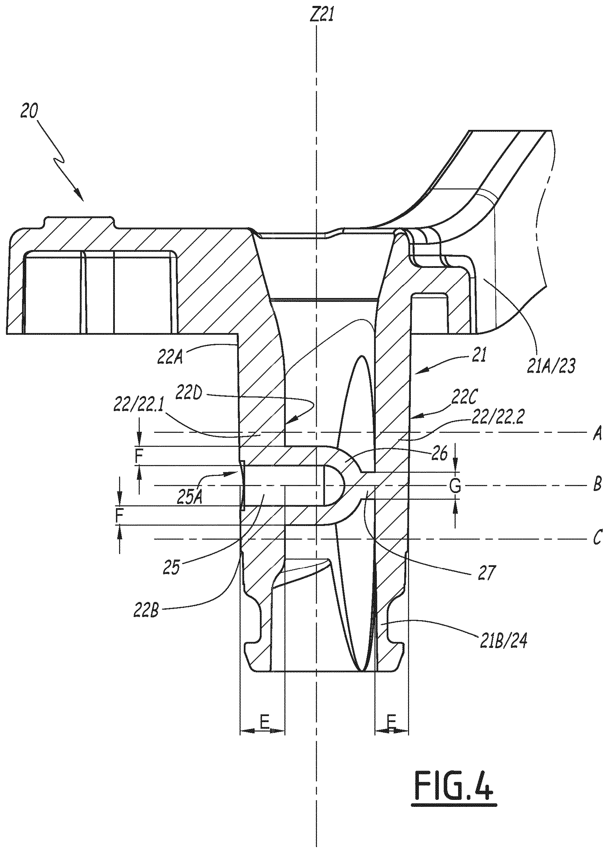

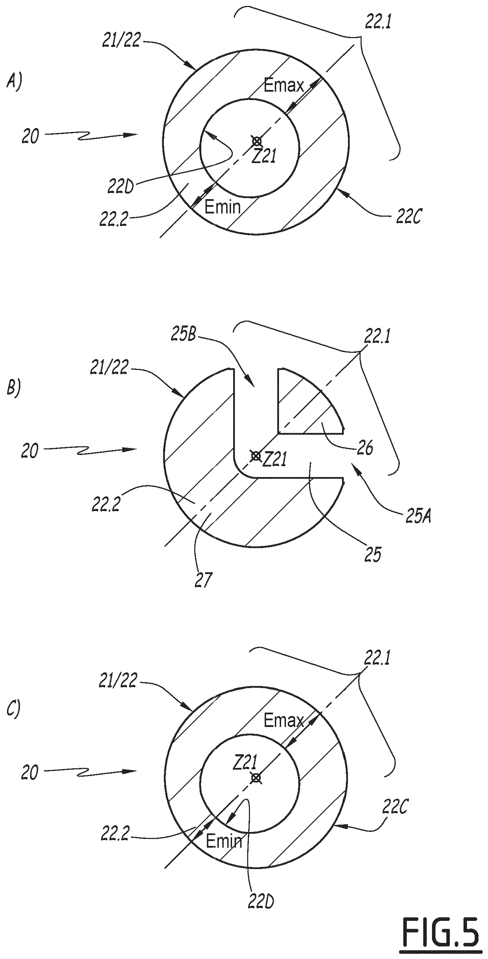

Le boisseau 20, qui est représenté seul sur les figures 3 à 5, comporte un fût 21. Comme bien visible sur les figures 4 et 5, le fût 21 est tubulaire, en étant centré sur un axe géométrique Z21.

À l'état assemblé du robinet médical 1, le fût 21 est monté sur le corps 10, en étant au moins partiellement reçu à l'intérieur du volume interne V11 tout en y étant déplaçable en rotation autour de l'axe Z21 par rapport au corps 10. A cet effet, le fût 21 inclut une paroi tubulaire 22, qui, à l'état assemblé du robinet médical 1, est intégralement logée à l'intérieur du volume interne V11 et qui, dans l'exemple de réalisation considéré sur les figures, forme la partie courante du fût 21 suivant l'axe Z21. De plus, suivant une disposition avantageuse, le fût 21 est, à l'une de ses deux parties terminales 21A et 21B qui sont axialement opposées l'une à l'autre suivant l'axe Z21, muni d'un moyen de préhension 23, ici un levier, permettant d'être saisi manuellement par un utilisateur aux fins de l'entraînement du boisseau 20 en rotation autour de l'axe Z21 par rapport au corps 10. En outre, suivant une disposition également avantageuse, le fût 21 est, à sa partie terminale 21B opposée à sa partie terminale 21A munie du moyen de préhension 23, muni d'un moyen de liaison et de maintien 24, ici une collerette, permettant de maintenir le fût 21 à l'intérieur du volume interne V11 et de lier fixement l'un à l'autre suivant l'axe Z21 le boisseau 20 et le corps 10, par exemple par clipsage ou par bouterollage. La paroi tubulaire 22 relie l'une à l'autre les parties terminales 21A et 21B du fût 21, en s'étendant d'une extrémité axiale 22A de cette paroi tubulaire 22 à une extrémité axiale 22B de cette paroi tubulaire 22, opposée à l'extrémité axiale 22A suivant l'axe Z21, ces extrémités axiales 22A et 22B étant respectivement tournées vers la partie terminale 21A et la partie terminale 21B du fût 21.

La paroi tubulaire 22 est pourvue d'une surface extérieure 22C qui est prévue complémentaire du volume interne V11 de manière à, à l'état assemblé du robinet médical 1, étancher le montage du fût 21 à l'intérieur du volume interne V11. Autour de l'axe Z21, cette surface extérieure 22C s'étend sur 360°. Suivant l'axe Z21, cette surface extérieure 22C s'étend sur toute l'étendue axiale de la paroi tubulaire 22, autrement dit de l'une à l'autre des deux extrémités axiales 22A et 22B. En pratique, cette surface extérieure 22C est cylindrique et/ou légèrement tronconique, en étant centrée sur l'axe Z21, les spécificités correspondantes de cette surface extérieure 22C n'étant pas limitatives du moment que la coopération par complémentarité de formes entre cette surface extérieure 22C et le volume interne V11 rend étanche le montage rotatif du fût 21 à l'intérieur de ce volume interne V11. En particulier, la surface extérieure 22C de la paroi tubulaire 22 présente avantageusement, en tout plan géométrique perpendiculaire Z21, un contour circulaire qui est centré sur cet axe Z21, comme bien visible sur les inserts A), B) et C) de la figure 5.

Eu égard à sa forme tubulaire, la paroi tubulaire 22 est également pourvue d'une surface intérieure 22D qui, à la différence de la surface extérieure 22C, est tournée radialement vers l'axe Z21. Autour de l'axe Z21, la surface intérieure 22D s'étend sur 360°. Suivant l'axe Z21, la surface intérieure 22D s'étend sur toute l'étendue axiale de la paroi tubulaire 22.

Le boisseau 20 comporte également un canal 25 qui est fixement solidaire du fût 21 et qui, en fonction de la position angulaire du fût 21 autour de l'axe Z21 par rapport au corps 10, met en communication entre eux au moins deux des ports 12 à 15 du corps 10. Autrement dit, le canal 25 commande la mise en communication entre les ports 12 à 15 en fonction de la position angulaire du boisseau 20 autour de l'axe Z21 par rapport au corps 10 : ainsi, lorsque le fût 21 occupe une position angulaire ad hoc, le canal 25 autorise à travers lui un écoulement entre au moins deux des ports 12 à 15, tout en isolant le ou les autres ports le cas échéant ; et lorsque le fût 21 occupe une autre position angulaire ad hoc, le canal 25 autorise à travers lui un écoulement entre au moins deux autres des ports 12 à 15, tout en isolant le ou les autres ports le cas échéant. Par exemple, la figure 2 montre bien que, dans la position angulaire du fût 21 considérée sur cette figure 2, le canal 25 met en communication entre eux les ports 12 et 13, tout en isolant les ports 14 et 15.

Le canal 25 traverse la paroi tubulaire 22 du fût 21 de part en part, c'est-à-dire depuis la surface extérieure 22C jusqu'à la surface intérieure 22D de la paroi tubulaire 22, et s'étend à l'intérieur du fût 21 où le canal 25 est délimité par une paroi canalaire 26 en saillie de la surface intérieure 22D. Le canal 25 débouche sur la surface extérieure 22C par au moins deux ouvertures distinctes, qui sont reliées entre elles par le canal 25 et qui sont réparties autour de l'axe Z21, en étant toutes situées dans une même portion 22.1 de la paroi tubulaire 22, cette portion 22.1 s'étendant autour de l'axe Z21 sur au plus 180° environ depuis l'une de ces ouvertures jusqu'à une autre de ces ouvertures, comme indiqué schématiquement sur l'insert B) de la figure 5. En fonction de la position angulaire du fût 21 autour de l'axe Z21 par rapport au corps 10, les ouvertures précitées sont alignables avec autant de ports respectifs parmi les ports 12 à 15 du corps 10. Dans le mode de réalisation considérée sur les figures 1 à 5, les ouvertures précitées sont au nombre de deux, en étant respectivement référencées 25A et 25B, de sorte que le canal 25 permet avantageusement de mettre en communication entre eux uniquement deux des ports 12 à 15, tout en isolant les deux autres ports, en fonction de la position angulaire du fût 21. De plus, également dans le mode de réalisation considéré sur les figures 1 à 5, la portion 22.1 de la paroi tubulaire 22 s'étend avantageusement sur 90° environ autour de l'axe Z21, comme illustré schématiquement sur les inserts A), B) et C) de la figure 5. Le canal 25 présente alors avantageusement une forme en L, comme bien visible sur la figure 2 et sur l'insert B) de la figure 5. Dans tous les cas, les ouvertures du canal 25, telles que les ouvertures 25A et 25B, sont toutes situées, suivant l'axe Z21, à la fois entre et à distance des extrémités axiales 22A et 22B de la paroi tubulaire 22 ; dans le mode de réalisation considéré sur les figures, les ouvertures 25A et 25B sont ainsi situées axialement à mi-distance des extrémités axiales 22A et 22B.

La paroi canalaire 26 s'étend, au moins pour partie, depuis la portion 22.1 de la paroi tubulaire 22, comme bien visible sur la figure 4 et sur l'insert B) de la figure 5. Dans la forme de réalisation considérée ici, la paroi canalaire 26 s'étend ainsi de la portion 22.1 de la paroi tubulaire 22, en étant saillante de la surface intérieure 22D de la paroi tubulaire 22 suivant une direction radiale à l'axe Z21.

Notamment afin de rigidifier la structure que forme la paroi canalaire 26 à l'intérieur du fût 21, le boisseau 20 comprend avantageusement une cloison 27 qui, comme bien visible sur la figure 4, s'étend perpendiculairement à l'axe Z21 et relie la paroi canalaire 26 et une portion 22.2 de la paroi tubulaire, diamétralement opposée à la portion 22.1.

Avant de décrire plus en détail certains aspects dimensionnels du boisseau 20, on notera que le fût 21, le canal 25 et la cloison 27 sont avantageusement réalisés d'une seule pièce, formant ici la totalité du boisseau 20, qui est réalisée en une matière plastique. Cette matière plastique est notamment façonnée par injection dans un moule. Dans tous les cas, la matière plastique constituant le boisseau 20 permet avantageusement à ce boisseau de se conformer, par légère déformation, au volume interne V11 du corps 10. La matière plastique du boisseau 20 est préférentiellement choisie parmi le polyéthylène (PE), le polypropylène (PP), le polyoxyméthylène (POM) et le polytéréphtalate de butylène (PBT), en étant encore plus préférentiellement choisie comme étant du polyoxyméthylène (POM).

Par ailleurs, la matière plastique du boisseau 20 est avantageusement différente du matériau constituant le fût 11 et, plus généralement, le corps 10, notamment afin d'améliorer les propriétés de rotation du boisseau 20 dans le corps 10. En particulier, le fût 11 est avantageusement réalisé en une matière plastique dont de nombreux exemples sont donnés dans WO2016/166346 auquel le lecteur peut se reporter. Le fût 11 est préférentiellement réalisé en polyamide ou en une matière plastique comprenant du polyamide.

Dans tous les cas, les matériaux constitutifs du corps 10 et du boisseau 20 supportent les contraintes mécaniques et chimiques auxquelles le robinet médical 1 est prévu d'être soumis. Les contraintes mécaniques sont essentiellement la déformation par cisaillement et la pression exercée sur le robinet médical 1 lors de sa fabrication et son utilisation. Les contraintes chimiques sont essentiellement liées aux produits prévus de circuler à l'intérieur du robinet médical 1 : en pratique, les matériaux précités sont résistants à tout produit pharmaceutique, y compris les produits huileux, en particulier au produit Lipiodol^{®}.

En revenant maintenant à la description de certains aspects dimensionnels du boisseau 20, les figures 4 et 5 montrent bien que la paroi tubulaire 22 ne présente pas une épaisseur constante autour de l'axe Z21. Plus précisément, la paroi tubulaire 22 est intérieurement plus épaisse dans sa portion 22.1 que dans le reste de la paroi tubulaire 22. Cela revient à dire que la paroi tubulaire 22 est pourvue d'une surépaisseur interne, localisée dans sa portion 22.1 comparativement au reste de la paroi tubulaire 22. Suivant l'axe Z21, cette surépaisseur interne s'étend de part et d'autre du canal 25, voire, comme ici, sur toute l'étendue axiale de la paroi tubulaire 22. Il en résulte que, comme bien visible sur les inserts A) et C) de la figure 5, la surface intérieure 22D de la paroi tubulaire 22 présente, axialement de part et d'autre du canal 25, un contour transversal, c'est-à-dire en tout plan perpendiculaire à l'axe Z21, qui est décentré par rapport à l'axe Z21.

Grâce à la surépaisseur interne de la paroi tubulaire 22 du fût 21, localisée dans la portion 22.1 de cette paroi tubulaire 22, la résistance aux fuites du robinet médical 1 est substantiellement renforcée. Sans vouloir être liés par une théorie, les inventeurs ont découvert que cette surépaisseur interne localisée tend à ce que la pression de contact au sein de l'interface entre la paroi tubulaire 22 et le volume interne V11 du fût 11 soit spécifiquement augmentée dans la portion 22.1 de la paroi tubulaire 22, et ce axialement de part et d'autre du canal 25, en formant ainsi respectivement deux barrières d'étanchéité qui permettent de maîtriser l'infiltration au sein de l'interface précitée et, par-là, de minimiser les fuites à l'extérieur de cette interface lors de l'utilisation du robinet médical 1, y compris à des pressions d'utilisation élevées, typiquement supérieures à 65 bars. Le renfort de la résistance aux fuites est ainsi localisé dans la zone du boisseau 20 sur laquelle s'exerce le plus de pression lors de l'utilisation du robinet médical 1, tout en répartissant le champ de pression avec la zone diamétralement opposée du boisseau 20. Dans le même temps, le couple nécessaire à la mise en rotation du boisseau 20 par rapport au corps 10 autour de l'axe Z21 ne s'en trouve pas significativement affecté.

Ci-après, on appelle « épaisseur de fût » la dimension de la paroi tubulaire 22, qui est radiale à l'axe Z21 et qui sépare l'une de l'autre les surfaces extérieure 22C et intérieure 22D de cette paroi tubulaire 22. Comme bien visible sur les figures 4 et 5 sur lesquelles l'épaisseur de fût est référencée E, cette épaisseur de fût E varie autour de l'axe Z21, avantageusement de manière continue, en étant plus grande dans la portion 22.1 que dans le reste de la paroi tubulaire 22. Ainsi, sur la figure 4, l'épaisseur de fût E présente des valeurs respectives différentes dans la moitié gauche et dans la moitié droite de cette figure. On notera que, selon les spécificités géométriques des surfaces extérieure 22C et intérieure 22D de la paroi tubulaire 22, l'épaisseur de fût E peut, en un point donné de la périphérie de la paroi tubulaire 22 et quelle que soit la position de ce point autour de l'axe Z21, ne pas être rigoureusement constante sur toute l'étendue axiale de la paroi tubulaire 22 ; toutefois, en un plan donné perpendiculaire à l'axe Z21 et quelle que soit la position de ce plan sur toute l'étendue axiale de la paroi tubulaire 22, l'épaisseur de fût E est plus grande dans la portion 22.1 que dans le reste de la paroi tubulaire 22, comme bien visible sur la figure 4.

Dans le mode de réalisation considéré aux figures 1 à 5, l'épaisseur de fût E varie autour de l'axe Z21, avantageusement de manière continue, entre un maximum Emax, qui est atteint en un premier point de la périphérie de la paroi tubulaire 22, situé dans la portion 22.1 et à mi-distance des ouvertures 25A et 25B autour de l'axe Z21, et un minimum Emin, qui est atteint en un second point de la périphérie de la paroi tubulaire 22, diamétralement opposé au premier point précité, comme indiqué sur la figure 5.

Suivant des dispositions optionnelles, qui sont mises en œuvre dans le mode de réalisation considéré sur les figures 1 à 5 et qui visent à renforcer davantage la résistance aux fuites pour le robinet médical 1, la paroi canalaire 26 et la cloison 27 présentent des dimensions spécifiques, détaillées ci-après.

On appelle « épaisseur de canal » la dimension axiale que présente la paroi canalaire 26 axialement de part et d'autre du canal 25. Cette épaisseur de canal est référencée F sur la figure 4. On appelle « épaisseur de cloison » la dimension axiale de la cloison 27. Cette épaisseur de cloison est référencée G sur la figure 4.

Selon une première disposition avantageuse, l'épaisseur de canal F est inférieure à la valeur minimale de l'épaisseur de fût E dans la portion 22.1 de la paroi tubulaire 22 et l'épaisseur de cloison G est inférieure à la valeur minimale de l'épaisseur de fût E dans la portion 22.2 de la paroi tubulaire 22. De cette façon, les phénomènes de retassures sont évités lors de la fabrication du boisseau 20. Bien entendu, l'épaisseur de canal F et l'épaisseur de cloison G doivent présenter une valeur minimale liée au fait que la matière plastique puisse remplir les cavités de moulage correspondantes, sans risquer de manquer de matière.

Selon une autre disposition avantageuse, l'épaisseur de cloison G est supérieure à l'épaisseur de canal F. De cette façon, on limite davantage la déformation de la paroi canalaire 26 perpendiculairement à l'axe Z21 lorsque le canal 25 est sous pression, c'est-à-dire lorsque s'écoule dans le canal 25 un fluide sous forte pression, typiquement supérieure à 65 bars.

Selon encore une autre disposition avantageuse, l'épaisseur de canal F est égale à 40%, plus ou moins 10%, de la valeur minimale de l'épaisseur de fût E dans la portion 22.1 de la paroi tubulaire, et l'épaisseur de cloison G est égale à 85%, plus ou moins 10%, de la valeur minimale de l'épaisseur de fût E dans la portion 22.2 de la paroi tubulaire 22. De cette façon, les inventeurs ont établi que les déformations du canal 25 sous pression sont maitrisées, en évitant à la fois que la paroi canalaire 26 ne se déforme significativement de manière transversale à l'axe Z21 et que les bordures des ouvertures 25A et 25B soient sensiblement déformées au niveau de l'interface entre la paroi tubulaire 22 et le volume interne V11 du fût 11.

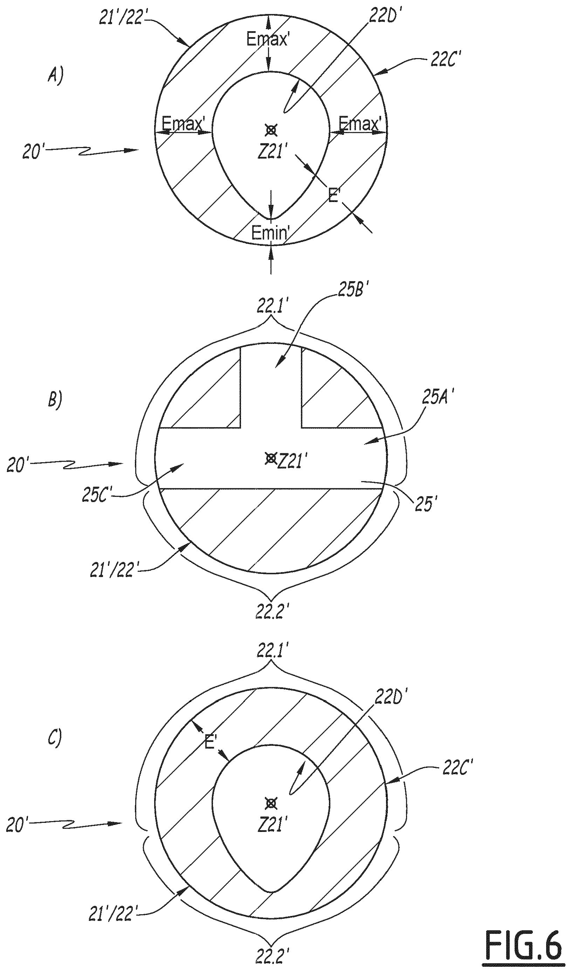

Comme évoqué plus haut, les ouvertures par lesquelles le canal 25 débouche sur la surface extérieure 22C de la paroi tubulaire 22 du fût 21, telles que les ouvertures 25A et 25B, peuvent être prévues en un nombre plus grand que deux. De même, l'étendue angulaire de la portion 22.1 de la paroi tubulaire 22 n'est pas limitée à 90° environ du moment que cette étendue angulaire reste inférieure à 180° environ. Il en résulte que, plutôt qu'être en forme de L, le canal 25 peut présenter de multiples autres formes géométriques. À titre d'exemple, cet aspect est illustré par la figure 6 qui montre un mode de réalisation alternatif pour le boisseau 20, qui est référencé 20'.

Le boisseau 20' est fonctionnellement similaire au boisseau 20, tout en s'en distinguant structurellement, comme détaillé ci-après. Le boisseau 20' comprend ainsi, d'une part, un fût 21', qui est fonctionnellement similaire au fût 21, notamment en étant centré sur un axe géométrique Z21', fonctionnellement similaire à l'axe Z21, et en incluant une paroi tubulaire 22' fonctionnellement similaire à la paroi tubulaire 22, et, d'autre part, un canal 25' qui est fonctionnellement similaire au canal 25, mais qui s'en distingue structurellement par le fait que le canal 25' débouche sur la surface extérieure 22C' de la paroi tubulaire 22' non pas par deux ouvertures, mais par trois ouvertures qui sont respectivement référencées 25A', 25B' et 25C', comme bien visible sur l'insert B) de la figure 6. Les ouvertures 25A', 25B' et 25C' sont reliées entre elles par le canal 25' et sont réparties autour de l'axe Z21', en étant toutes situées dans une même portion 22.1' de la paroi tubulaire 22'. La portion 22.1' de la paroi tubulaire 22' est fonctionnellement similaire à la portion 22.1 de la paroi tubulaire 22, mais s'en distingue structurellement par le fait que la portion 22.1' s'étend sur 180° environ. Ici, les ouvertures 25A', 25B' et 25C' sont reparties autour de l'axe Z21' de manière sensiblement régulière, si bien que le canal 25' présente une forme en T, comme bien visible sur l'insert B) de la figure 6.

Suivant des considérations similaires à celles détaillées plus haut pour le boisseau 20, la paroi tubulaire 22' du fût 21' du boisseau 20' est intérieurement plus épaisse dans sa portion 22.1' que dans le reste de la paroi tubulaire 22', comme bien visible sur la figure 6. L'épaisseur de fût, notée E', du fût 21' varie ainsi autour de l'axe Z21', avantageusement de manière continue, en étant plus grande dans la portion 22.1' que dans le reste de la paroi tubulaire 22'.

Dans le mode de réalisation de la figure 6, l'épaisseur de fût E' varie ainsi autour de l'axe Z21', avantageusement de manière continue, entre :
- un maximum Emax', qui est atteint et sensiblement maintenu sur toute la portion 22.1' autour de l'axe Z21', et
- un minimum Emin', qui est atteint en un point de la périphérie de la paroi tubulaire 22', ce point étant situé, autour de l'axe Z21', sensiblement au milieu d'une portion 22.2' de la paroi tubulaire 22', qui est complémentaire de la portion 22.1' de cette dernière, comme bien visible sur les inserts A) et C) de la figure 6.

Il en résulte que, comme bien visible sur les inserts A) et C) de la figure 6, la surface intérieure 22D' de la paroi tubulaire 22' présente, axialement de part et d'autre du canal 25', un contour transversal en forme d'œuf, orientée vers le point de la périphérie de la paroi tubulaire 22', qui est associé au minimum Emin'.

Par ailleurs, bien que non reprises ici en détail, les considérations optionnelles détaillées plus haut pour le boisseau 20 en lien avec la paroi canalaire 26 et la cloison 27 s'appliquent, mutatis mutandis, au boisseau 20'.

Enfin, divers aménagements et variantes au robinet médical 1 décrit jusqu'ici sont par ailleurs envisageables. À titre d'exemples :
- l'amplitude du mouvement rotatif du boisseau 20 ou 20' par rapport au corps 10 peut être limitée par des aménagements ad hoc du robinet médical 1 afin d'empêcher la mise en communication entre certains des ports 12 à 15, comme détaillé par exemple dans WO 2016/166346 auquel le lecteur peut se reporter ; le robinet médical 1 pourvu des quatre ports 12 à 15 peut ainsi notamment être conçu en tant que robinet à seulement trois voies ; et/ou
- le nombre de ports, tels que les ports 12 à 15, n'est pas limité à quatre, mais peut être égal à trois ou bien égal ou supérieur à cinq, chacun de ces différents ports pouvant former indifféremment un connecteur femelle ou un connecteur mâle.

## Revendications

1. Robinet médical (1), comportant :
- un corps (10), qui définit un volume interne (V11) et qui est muni d'au moins trois ports (12, 13, 14, 15) qui relient chacun le volume interne avec l'extérieur du corps, et
- un boisseau (20 ; 20') qui comprend :
- un fût (21 ; 21'), qui est tubulaire, en étant centré sur un axe (Z21 ; Z21'), et qui inclut une paroi tubulaire (22 ; 22') pourvue d'une surface extérieure (22C ; 22C') qui est complémentaire du volume interne (V11) de manière que le fût est reçu de manière étanche à l'intérieur du volume interne tout en y étant déplaçable en rotation autour de l'axe par rapport au corps (10), et
- un canal (25 ; 25') qui met en communication entre eux au moins deux des ports (12, 13, 14, 15) en fonction de la position angulaire du fût (21 ; 21') autour de l'axe (Z21 ; Z21') par rapport au corps (10), lequel canal traverse la paroi tubulaire (22 ; 22'), de la surface extérieure (22C ; 22C') à une surface intérieure (22D ; 22D') de la paroi tubulaire, et s'étend à l'intérieur du fût de manière à relier entre elles au moins deux ouvertures (25A, 25B ; 25A', 25B', 25C') par lesquelles le canal débouche sur la surface extérieure de la paroi tubulaire, ces ouvertures étant distinctes les unes des autres et étant toutes situées dans une même première portion (22.1 ; 22.1') de la paroi tubulaire, qui s'étend autour de l'axe sur au plus 180° environ depuis l'une des ouvertures jusqu'à une autre des ouvertures,
**caractérisé en ce que** la paroi tubulaire (22 ; 22') est, axialement de part et d'autre du canal (25 ; 25'), intérieurement plus épaisse dans sa première portion (22.1 ; 22.1') que dans le reste de la paroi tubulaire.

2. Robinet médical suivant la revendication 1, dans lequel uniquement deux ouvertures (25A, 25B) sont prévues.

3. Robinet médical suivant l'une des revendications 1 ou 2, dans lequel la première portion (22.1) de la paroi tubulaire (22) s'étend autour de l'axe (Z21) sur 90° environ.

4. Robinet médical suivant la revendication 2, dans lequel la première portion (22.1) de la paroi tubulaire (22) s'étend autour de l'axe (Z21) sur 90° environ, et dans lequel le canal (25) présente une forme en L.

5. Robinet médical suivant la revendication 1, dans lequel trois ouvertures (25A', 25B', 25C') sont prévues.

6. Robinet médical suivant l'une des revendications 1 ou 5, dans lequel la première portion (22.1') de la paroi tubulaire (22') s'étend autour de l'axe (Z21') sur 180° environ.

7. Robinet médical suivant l'une quelconque des revendications précédentes,
dans lequel la surface extérieure (22C ; 22C') de la paroi tubulaire (22 ; 22') présente, en tout plan perpendiculaire à l'axe (Z21 ; Z21'), un contour circulaire qui est centré sur l'axe, et dans lequel la paroi tubulaire (22 ; 22') présente une dimension radiale à l'axe (Z21 ; Z21'), appelée épaisseur de fût (E ; E'), qui sépare l'une de l'autre les surfaces extérieure (22C ; 22C') et intérieure (22D ; 22D') de la paroi tubulaire, et qui varie autour de l'axe, en étant plus grande dans la première portion (22.1 ; 22.1') que dans le reste de la paroi tubulaire.

8. Robinet médical suivant l'une des revendications 2 ou 4,
dans lequel la surface extérieure (22C) de la paroi tubulaire (22) présente, en tout plan perpendiculaire à l'axe (Z21), un contour circulaire qui est centré sur l'axe,
dans lequel la paroi tubulaire (22) présente une dimension radiale à l'axe (Z21), appelée épaisseur de fût (E), qui sépare l'une de l'autre les surfaces extérieure (22C) et intérieure (22D) de la paroi tubulaire, et qui varie autour de l'axe, en étant plus grande dans la première portion (22.1) que dans le reste de la paroi tubulaire,
et dans lequel l'épaisseur de fût (E) varie autour de l'axe (Z21) entre :
- un maximum (Emax) qui est atteint en un premier point de la périphérie de la paroi tubulaire (22), situé dans la première portion (22.1) et à sensiblement mi-distance des deux ouvertures (25A, 25B) autour de l'axe (Z21), et
- un minimum (Emin) qui est atteint en un second point de la périphérie de la paroi tubulaire, diamétralement opposé au premier point.

9. Robinet médical suivant la revendication 5,
dans lequel la surface extérieure (22C') de la paroi tubulaire (22') présente, en tout plan perpendiculaire à l'axe (Z21'), un contour circulaire qui est centré sur l'axe,
dans lequel la paroi tubulaire (22') présente une dimension radiale à l'axe (Z21'), appelée épaisseur de fût (E'), qui sépare l'une de l'autre les surfaces extérieure (22C') et intérieure (22D') de la paroi tubulaire, et qui varie autour de l'axe, en étant plus grande dans la première portion (22.1') que dans le reste de la paroi tubulaire,
et dans lequel l'épaisseur de fût (E') varie autour de l'axe (Z21') entre :
- un maximum (Emax') qui est atteint et sensiblement maintenu sur toute la première portion (22.1') autour de l'axe (Z21'), et
- un minimum (Emin') qui est atteint en un point de la périphérie de la paroi tubulaire (22'), ce point étant situé, autour de l'axe (Z21'), sensiblement au milieu d'une portion (22.2') de la paroi tubulaire, qui est complémentaire de la première portion (22.1').

10. Robinet médical suivant l'une quelconque des revendications 7 à 9, dans lequel l'épaisseur de fût (E ; E') varie autour de l'axe (Z21 ; Z21') de manière continue.

11. Robinet médical suivant l'une quelconque des revendications 7 à 10, dans lequel le canal (25 ; 25') est, à l'intérieur du fût (21 ; 21'), délimité par une paroi canalaire (26) qui s'étend depuis la première portion (22.1 ; 22.1') de la paroi tubulaire (22 ; 22'), en étant radialement saillante de la surface intérieure (22D ; 22D') de la paroi tubulaire, dans lequel la paroi canalaire (26) présente, axialement de part et d'autre du canal (25 ; 25'), une dimension axiale, appelée épaisseur de canal (F), qui est inférieure à la valeur minimale de l'épaisseur de fût (E ; E') dans la portion (22.1 ; 22.1') de la paroi tubulaire (22 ; 22'),
dans lequel le boisseau (20 ; 20') comprend également une cloison (27), qui s'étend perpendiculairement à l'axe (Z21 ; Z21') et qui relie la paroi canalaire (26) et une seconde portion (22.2 ; 22.2') de la paroi tubulaire (22 ; 22'), qui est diamétralement opposée à la première portion (22.1 ; 22.1') de la paroi tubulaire,
et dans lequel la cloison (27) présente une dimension axiale, appelée épaisseur de cloison (G), qui est inférieure à la valeur minimale de l'épaisseur de fût (E ; E') dans la seconde portion (22.2 ; 22.1') de la paroi tubulaire (22 ; 22').

12. Robinet médical suivant la revendication 11, dans lequel l'épaisseur de cloison (G) est supérieure à l'épaisseur de canal (F).

13. Robinet médical suivant l'une des revendications 11 ou 12,
dans lequel l'épaisseur de canal (F) est égale à 40%, plus ou moins 10%, de la valeur minimale de l'épaisseur de fût (E ; E') dans la première portion (22.1 ; 22.1') de la paroi tubulaire (22 ; 22'),
et dans lequel l'épaisseur de cloison (G) est égale à 85%, plus ou moins 10%, de la valeur minimale de l'épaisseur de fût (E ; E') dans la seconde portion (22.2 ; 22.2') de la paroi tubulaire (22 ; 22').

## Patentansprüche

1. Medizinisches Ventil (1), umfassend:
- einen Körper (10), der ein Innenvolumen (V11) definiert und der mit mindestens drei Anschlüssen (12, 13, 14, 15) versehen ist, die jeweils das Innenvolumen mit der Außenseite des Körpers verbinden, und - ein Küken (20; 20'), das Folgendes umfasst:
- einen Schaft (21; 21'), der rohrförmig ist, indem er auf einer Achse (Z21; Z21') zentriert ist, und der eine rohrförmige Wand (22; 22') mit einer Außenfläche (22C; 22C') beinhaltet, die komplementär zu dem Innenvolumen (V11) ist, sodass der Schaft dicht in dem Innenvolumen aufgenommen ist, während er darin um die Achse in Bezug auf den Körper (10) drehbar ist, und
- einen Kanal (25; 25'), der mindestens zwei der Öffnungen (12, 13, 14, 15) abhängig von der Winkelposition des Schafts (21; 21') um die Achse (Z21; Z21') in Bezug auf den Körper (10) miteinander verbindet, wobei der Kanal durch die rohrförmige Wand (22; 22') von der Außenfläche (22C; 22C') zu einer Innenfläche (22D ; 22D') der rohrförmigen Wand verläuft, und sich innerhalb des Schafts erstreckt, um mindestens zwei Öffnungen (25A, 25B; 25A', 25B', 25C') miteinander zu verbinden, durch die der Kanal in die Außenfläche der rohrförmigen Wand mündet, wobei diese Öffnungen voneinander verschieden sind und sich alle in demselben ersten Abschnitt (22.1; 22.1') der rohrförmigen Wand befinden, der sich um die Achse über höchstens etwa 180° von einer der Öffnungen zu einer anderen der Öffnungen erstreckt, **dadurch gekennzeichnet, dass** die rohrförmige Wand (22; 22') axial auf beiden Seiten des Kanals (25; 25') in ihrem ersten Abschnitt (22.1; 22.1') innen stärker ist als im Rest der rohrförmigen Wand.

2. Medizinisches Ventil nach Anspruch 1, wobei nur zwei Öffnungen (25A, 25B) bereitgestellt sind.

3. Medizinisches Ventil nach einem der Ansprüche 1 oder 2, wobei sich der erste Abschnitt (22.1) der rohrförmigen Wand (22) um die Achse (Z21) um etwa 90° erstreckt.

4. Medizinisches Ventil nach Anspruch 2, wobei sich der erste Abschnitt (22.1) der rohrförmigen Wand (22) um die Achse (Z21) um etwa 90° erstreckt, und wobei der Kanal (25) eine L-Form aufweist.

5. Medizinisches Ventil nach Anspruch 1, wobei drei Öffnungen (25A', 25B', 25C') bereitgestellt sind.

6. Medizinisches Ventil nach einem der Ansprüche 1 oder 5, wobei sich der erste Abschnitt (22.1') der rohrförmigen Wand (22') um die Achse (Z21') um etwa 180° erstreckt.

7. Verfahren nach einem der vorherigen Ansprüche,
wobei die Außenfläche (22C; 22C') der rohrförmigen Wand (22; 22') in jeder Ebene senkrecht zu der Achse (Z21; Z21') eine kreisförmige Kontur aufweist, die auf der Achse zentriert ist, und wobei die rohrförmige Wand (22; 22') eine Abmessung radial zu der Achse (Z21; Z21'), die als Schaftstärke (E; E') bezeichnet wird, aufweist, die die Außenfläche (22C; 22C') und die Innenfläche (22D; 22D') der rohrförmigen Wand voneinander trennt und die um die Achse variiert, wobei sie in dem ersten Abschnitt (22.1')größer ist als im Rest der rohrförmigen Wand.

8. Medizinisches Ventil nach einem der Ansprüche 2 oder 4, wobei die Außenfläche (22C) der rohrförmigen Wand (22) in jeder Ebene senkrecht zu der Achse (Z21) eine kreisförmige Kontur aufweist, die auf der Achse zentriert ist, wobei die rohrförmige Wand (22) eine Abmessung radial zu der Achse (Z21) aufweist, die als Schaftstärke (E) bezeichnet wird, die die Außenfläche (22C) und die Innenfläche (22D) der rohrförmigen Wand voneinander trennt, und die um die Achse variiert, wobei sie in dem ersten Abschnitt (22.1) größer als im Rest der Rohrwand ist, und wobei die Schaftstärke (E) um die Achse (Z21) variiert zwischen:
- einem Maximum (Emax), das an einem ersten Punkt des Umfangs der rohrförmigen Wand (22) erreicht wird, der sich in dem ersten Abschnitt (22.1) und im Wesentlichen in der Mitte zwischen den zwei Öffnungen (25A, 25B) um die Achse (Z21) befindet, und
- einem Minimum (Emin), das an einem zweiten Punkt des Umfangs der rohrförmigen Wand diametral gegenüber dem ersten Punkt erreicht wird.

9. Medizinisches Ventil nach Anspruch 5, wobei die Außenfläche (22C') der rohrförmigen Wand (22') in jeder Ebene senkrecht zu der Achse (Z21') eine kreisförmige Kontur aufweist, die auf der Achse zentriert ist, wobei die rohrförmige Wand (22') eine Abmessung radial zu der Achse (Z21') aufweist, die als Schaftstärke (E') bezeichnet wird, die die Außenfläche (22C') und die Innenfläche (22D') der rohrförmigen Wand voneinander trennt, und die um die Achse herum variiert, wobei sie in dem ersten Abschnitt (22.1') größer ist als im Rest der rohrförmigen Wand, und wobei die Schaftstärke (E') um die Achse (Z21') variiert zwischen:
- einem Maximum (Emax'), das über den gesamten ersten Abschnitt (22.1') um die Achse (Z21') erreicht und im Wesentlichen aufrechterhalten wird, und
- einem Minimum (Emin'), das an einem Punkt am Umfang der rohrförmigen Wand (22') erreicht wird, wobei sich dieser Punkt um die Achse (Z21') herum im Wesentlichen in der Mitte eines Abschnitts (22.2') der rohrförmigen Wand befindet, der komplementär zu dem ersten Abschnitt (22.1') ist.

10. Medizinisches Ventil nach einem der Ansprüche 7 bis 9, wobei die Schaftstärke (E; E') um die Achse (Z21; Z21') kontinuierlich variiert.

11. Medizinisches Ventil nach einem der Ansprüche 7 bis 10, wobei der Kanal (25; 25') innerhalb des Schafts (21; 21') durch eine Kanalwand (26) begrenzt ist, die sich von dem ersten Abschnitt (22.1; 22.1') der rohrförmigen Wand (22; 22') erstreckt, indem sie radial von der Innenfläche (22D; 22D') der rohrförmigen Wand hervorsteht, wobei die Kanalwand (26) axial auf beiden Seiten des Kanals (25; 25') eine axiale Abmessung, die als Kanalstärke (F) bezeichnet wird, aufweist, die geringer ist als der Minimalwert der Schaftstärke (E; E') in dem Abschnitt (22.1; 22.1') der rohrförmigen Wand (22; 22'),
wobei das Küken (20; 20') auch eine Trennwand (27) umfasst, die sich senkrecht zu der Achse (Z21; Z21') erstreckt und die Kanalwand (26) und einen zweiten Abschnitt (22.2; 22.2') der rohrförmigen Wand (22; 22') verbindet, der dem ersten Abschnitt (22.1; 22.1') der rohrförmigen Wand diametral gegenüberliegt,
und wobei die Trennwand (27) eine axiale Abmessung aufweist, die als Trennwandstärke (G) bezeichnet wird, die kleiner ist als der Minimalwert der Schaftstärke (E; E') in dem zweiten Abschnitt (22.2; 22.1') der rohrförmigen Wand (22; 22').

12. Medizinisches Ventil nach Anspruch 11, wobei die Stärke der Trennwand (G) größer ist als die Stärke des Kanals (F).

13. Medizinisches Ventil nach einem der Ansprüche 11 oder 12, wobei die Kanalstärke (F) 40 %, plus oder minus 10 %, des Minimalwerts der Schaftstärke (E; E') in dem ersten Abschnitt (22.1; 22.1') der rohrförmigen Wand (22; 22') ist,
und wobei die Trennwandstärke (G) 85 %, plus oder minus 10 %, des Minimalwerts der Schaftstärke (E; E') in dem zweiten Abschnitt (22.2; 22.2') der rohrförmigen Wand (22; 22') ist.

## Claims

1. A medical valve (1), comprising:
- a body (10), which defines an internal volume (V11) and which is provided with at least three ports (12, 13, 14, 15) which each connect the internal volume with the outside of the body, and - a plug (20; 20') which comprises:
- a barrel (21; 21'), which is tubular, being centred on an axis (Z21; Z21'), and which includes a tubular wall (22; 22') provided with an outer surface (22C; 22C') which is complementary to the internal volume (V11) so that the barrel is sealingly received inside the internal volume while being rotatable therein about the axis with respect to the body (10), and
- a channel (25; 25') which connects together at least two of the ports (12, 13, 14, 15) as a function of the angular position of the barrel (21; 21') about the axis (Z21; Z21') relative to the body (10), which channel passes through the tubular wall (22; 22') from the outer surface (22C; 22C') to an inner surface (22D ; 22D') of the tubular wall, and extends inside the barrel so as to interconnect at least two openings (25A, 25B; 25A', 25B', 25C') through which the channel emerges onto the outer surface of the tubular wall, these openings being distinct from one another and all being located in the same first portion (22.1; 22.1') of the tubular wall, which extends about the axis over at most approximately 180° from one of the openings to another of the openings, **characterised in that** the tubular wall (22; 22') is, axially on either side of the channel (25; 25'), internally thicker in its first portion (22.1; 22.1') than in the rest of the tubular wall.

2. The medical valve according to claim 1, wherein only two openings (25A, 25B) are provided.

3. The medical valve according to one of claims 1 or 2, wherein the first portion (22.1) of the tubular wall (22) extends about the axis (Z21) for approximately 90°.

4. The medical valve according to claim 2, wherein the first portion (22.1) of the tubular wall (22) extends about the axis (Z21) by approximately 90°, and wherein the channel (25) is L-shaped.

5. The medical valve according to claim 1, wherein three openings (25A', 25B', 25C') are provided.

6. The medical valve according to one of claims 1 or 5, wherein the first portion (22.1') of the tubular wall (22') extends about the axis (Z21') over approximately 180°.

7. The medical valve according to any one of the preceding claims,
wherein the outer surface (22C; 22C') of the tubular wall (22; 22') has, in any plane perpendicular to the axis (Z21; Z21') a circular contour which is centred on the axis, and wherein the tubular wall (22; 22') has a dimension radial to the axis (Z21; Z21') called the barrel thickness (E; E'), which separates the outer (22C; 22C') and inner (22D; 22D') surfaces of the tubular wall from one another; and which varies about the axis, being greater in the first portion (22.1; 22.1') than in the rest of the tubular wall.

8. The medical valve according to one of claims 2 or 4, wherein the outer surface (22C) of the tubular wall (22) has, in any plane perpendicular to the axis (Z21), a circular contour which is centred on the axis, wherein the tubular wall (22) has a dimension radial to the axis (Z21), called the barrel thickness (E), which separates the outer (22C) and inner (22D) surfaces of the tubular wall from each other, and which varies about the axis, being greater in the first portion (22.1) than in the rest of the tubular wall, and wherein the barrel thickness (E) varies about the axis (Z21) between:
- a maximum (Emax) which is reached at a first point on the periphery of the tubular wall (22), located in the first portion (22.1) and approximately halfway between the two openings (25A, 25B) about the axis (Z21), and
- a minimum (Emin) which is reached at a second point on the periphery of the tubular wall, diametrically opposite the first point.

9. The medical valve according to claim 5, wherein the outer surface (22C') of the tubular wall (22') has, in any plane perpendicular to the axis (Z21'), a circular contour which is centred on the axis, wherein the tubular wall (22') has a dimension radial to the axis (Z21') called the barrel thickness (E'), which separates the outer (22C') and inner (22D') surfaces of the tubular wall from each other, and which varies about the axis, being greater in the first portion (22.1') than in the rest of the tubular wall, and wherein the barrel thickness (E') varies about the axis (Z21') between:
- a maximum (Emax') which is reached and substantially maintained over the entire first portion (22.1') about the axis (Z21'), and
- a minimum (Emin') which is reached at a point on the periphery of the tubular wall (22'), this point being located, about the axis (Z21'), substantially in the middle of a portion (22.2') of the tubular wall, which is complementary to the first portion (22.1').

10. The medical valve according to any one of claims 7 to 9, wherein the thickness of the barrel (E; E') varies continuously about the axis (Z21; Z21').

11. The medical valve according to any one of claims 7 to 10, wherein the channel (25; 25') is, inside the barrel (21; 21'), delimited by a channel wall (26) which extends from the first portion (22.1; 22.1') of the tubular wall (22; 22'), projecting radially from the inner surface (22D; 22D') of the tubular wall, wherein the channel wall (26) has, axially on either side of the channel (25; 25'), an axial dimension, called the channel thickness (F), which is less than the minimum value of the barrel thickness (E; E') in the portion (22.1; 22.1') of the tubular wall (22; 22'),
wherein the plug (20; 20') further comprises a partition (27), which extends perpendicularly to the axis (Z21; Z21') and which connects the channel wall (26) and a second portion (22.2; 22.2') of the tubular wall (22; 22'), which is diametrically opposite to the first portion (22.1; 22.1') of the tubular wall,
and wherein the partition (27) has an axial dimension, referred to as the partition thickness (G), which is less than the minimum value of the barrel thickness (E; E') in the second portion (22.2; 22.1') of the tubular wall (22; 22').

12. The medical valve according to claim 11, wherein the partition thickness (G) is greater than the channel thickness (F).

13. The medical valve according to any one of claims 11 or 12, wherein the channel thickness (F) is equal to 40%, plus or minus 10%, of the minimum value of the barrel thickness (E; E') in the first portion (22.1; 22.1') of the tubular wall (22; 22'),
and wherein the partition thickness (G) is equal to 85%, plus or minus 10%, of the minimum value of the barrel thickness (E; E') in the second portion (22.2; 22.2') of the tubular wall (22; 22').
